# EUROPEAN PATENT SPECIFICATION

(11) **EP 3 836 856 B1**
(45) Date of publication and mention of the grant of the patent: **27.11.2024**
(21) Application number: 19849078.1
(22) Date of filing: 15.08.2019
(51) Int. Cl.: A61B 17/3213, A61D 1/06, A61D 7/00, A61M 11/00, A61M 19/00

(54) **DEVICE FOR DELIVERING LIQUIDS DURING SURGERY**
VORRICHTUNG ZUR ABGABE VON FLÜSSIGKEITEN WÄHREND EINER OPERATION
DISPOSITIF DE DISTRIBUTION DE LIQUIDES PENDANT UNE INTERVENTION CHIRURGICALE

(30) Priority: 15.08.2018 AU 2018217263
(43) Date of publication of application: 23.06.2021
(73) Proprietor: Larboard Pty Ltd, Braitling, Northern Territory 0870 (AU)
(72) Inventor: CHRISTIAN, James, Braitling, Northern Territory 0870 (AU)
(74) Representative: KIPA AB
(86) International application number: PCT/AU2019/050856
(87) International publication number: WO 2020/034005

(56) References cited:
- EP-A1- 2 248 550
- WO-A1-2008/148139
- WO-A1-2014/210149
- WO-A2-01/60235
- CN-A- 107 174 313
- CN-A- 107 951 543
- CN-U- 205 458 932
- US-A- 3 786 814
- US-A1- 2002 177 846
- US-A1- 2002 177 846
- US-A1- 2011 098 634
- US-A1- 2011 098 634
- US-A1- 2013 096 487

## Description

### Technical Field

The present disclosure relates to the delivery of fluids associated with surgery, for animals and humans, for example anaesthetics and antiseptics.

### Background of the Invention

During some surgical procedures, it is required or desired that a liquid of some kind is delivered shortly before, during or after the procedure, at or around the site of a surgical incision.

For example, veterinary castration procedures are carried out on cattle and other livestock. In order to meet modern demands for increasing animal welfare, it is known to utilise a spray of a surgical liquid which contains at least an anaesthetic, and desirably other components such as an antiseptic and potentially other components. An example of such a surgical liquid is the Tri-Solfen product available commercially from Bayer Australia, https://www.bayer.com.au/products/product-details.php?id=427.

The conventional process for castration using such a product is to first make an incision in the scrotum using a scalpel. The surgical liquid is then sprayed into the scrotum and onto the incision site using a spray head, typically fed by a reservoir of the liquid. The final incision to cut the spermatic cord is then completed and the testis removed. This is repeated for each testis.

The spray head is typically a pump action type device, operated by either the person performing the castration, or another person. It will be appreciated that as the procedure is being performed, for example in an outdoor situation, it will likely not be convenient or practical for the person to place the scalpel down and then grasp the spray head. The person may need to hold the animal with their other hand, particularly, as the procedure is carried out on an unsedated animal, and the animal will potentially move, kick, and so forth, making it difficult to manage the procedure with both hands occupied.

Some disclosures of the prior patent literature disclose devices for delivering liquids during surgery.

For example, WO 2001/060235 A2 by Thomas Fogarty discloses administering an anaesthetic agent such as lidocaine gel or liquid or the like to tissue through a distal end of tubular member via a syringe connected to a blade at a hub.

US 2002/0177846 A1 by Peter Mulier discloses a surgical device that is configured to emit vaporous medium (e.g. water vapour, or gaseous forms of other substances and may be loaded with additional substances) to a tissue site. The surgical device integrates other surgical components such as forceps, blades, snares or the like.

US 2013/0096487 A1 by Tyler Devin Panian discloses a device for applying medical liquid to an area and for cutting within the area. The device has an inlet port, a liquid flow channel, an outlet and a blade.

US 2011/0098634 A1 by Michael Wycoki discloses a topical anaesthetic and antiseptic dispensing device that has a penetrating instrument such as a scalpel and a connector arm affixed to a spray housing.

CN 107951543 A by Lishui Peoples Hospital discloses a scalpel with an integral blade. It also discloses a knife body that is used for surgery and a mechanism for injecting an anti-inflammatory drug through a liquid storage tube and an injection head to the skin incision.

CN205458932U by Jia Yunfeng discloses a surgical knife for neurosurgery that has a cutter body and a handle of a knife.

CN107174313A by Li Maolei discloses a scalpel for neurosurgery that has a knife and a blade.

EP 2248550 A1 by Koninklijke Philips Electronics NV discloses a surgical device that has a grip for holding the surgical device, a surgical tool element for use in surgical operations, a drug reservoir for comprising a drug, at least one drug delivery port and an actuator for delivering the drug from the drug reservoir.

US 3,786,814 by Thomas A. Armao discloses a method of preventing cryoadhesion of cryosurgical instruments comprises placing on the surface of the instrument which comes in contact with tissue a coating of a fluorocarbon anti-cryoadhesion material which is a good heat conducting material which is liquid at ambient temperatures and remains a liquid at cryogenic temperatures and which is nontoxic to tissue.

WO 2008/148139 A by Angelo Troedhan discloses a new approach to cutting by projecting sonic/ultrasonic oscillation energy via conventional scalpel blades and/or especially shaped thin and light steel and/or ceramic blades to organic cells, organic and non-organic fibres and/or fibre-tissue with or without a cooling irrigation by various liquids transported to the cutting site by an internal and/or external irrigation channel in or to the device.

However, none of these disclosures provide a device and method for delivering a surgical liquid spray during surgery which is simpler and more convenient for the operator.

It is an object of the present invention to provide a device for delivering a surgical liquid spray during a surgical procedure which is more convenient for the operator.

### Summary of the Invention

In a first broad form, the present invention provides a scalpel body which incorporates a spray head, so that a liquid spray may be delivered adjacent to a blade.

According to one aspect, the present invention provides a scalpel body as defined in claim 1. Further embodiments are recited in the dependent claims.

In suitable implementations of the present disclosure, a surgeon or other user may conveniently spray a surgical liquid from the spray head in the scalpel body, using one hand and without any need to adjust or separately aim a separate spray device. The implementations are cost effective as blades may be replaced as required, while the body is re-used. Thus, procedures may be made more convenient and precise in their use of surgical fluids.

### Brief Description of the Drawings

One implementation of the present invention will now be described with reference to the accompanying figures, in which:
Figure 1 is a conceptual side view of an implementation of the present invention;
Figure 2 is an isometric front view of the implementation of figure 1; and
Figure 3 is an isometric rear view of the implementation of figure 1; and
Figure 4A, B and C illustrate a blade connection procedure for an implementation of the present invention

### Detailed Description of the invention

The present invention will be described with reference to a specific implementation, having particular application to veterinary procedures. However, it will be appreciated that this example is illustrative and not limitative of the scope and field of application of the present invention. The present disclosure is applicable to any form or animal or human surgery is which it is desired to spray a medical liquid.

The present disclosure is particularly concerned with the delivery of surgical liquids. This term is intended to be understood broadly, as encompassing liquids intended to be delivered before, during and after surgery. These may include one or more components, including without limitation antiseptic agents, anaesthetics, carriers, hemostatic agents, and other materials. It is emphasised that the present disclosure is not specific to the actual fluid which is delivered, but is rather concerned with the mechanical systems to facilitate the delivery of such liquids. For example, one suitable product is the above referenced Tri-Solfen product, a gel containing Lignocaine (as hydrochloride) 40.6g/L, Bupivacaine (as hydrochloride) 4.2g/L, Adrenaline (as tartrate) 24.8mg/L, and Cetrimide 5g/L.

The surgical liquid is discussed as coming from a supply of liquid. This may be, for example, a container supplied by the product supplier, with a tube connecting the device to the contents. Other options include an infusion bag, elevated in use; a backpack type reservoir; a drum or other container, or any other suitable arrangement. Whilst the illustrated example is not pressurized, the present invention could be implemented using a pressurised system, either by a propellant or a pumped pressurisation technique (i.e. as in some agricultural sprayers).

The present implementation is intended to be used with disposable scalpel blades, and to operate as a body to retain such blades so that they can function as a scalpel. Of course, the present invention could in principle be used with re-usable blades if desired, but for the primary application described for the present implementation, blades become blunt from use. Any suitable blades may be used, with a corresponding mount compatible with the disposable blades being provided. It will be appreciated that the term scalpel is used in a broad sense, and encompasses any surgical instrument for cutting or penetrating an animal or person.

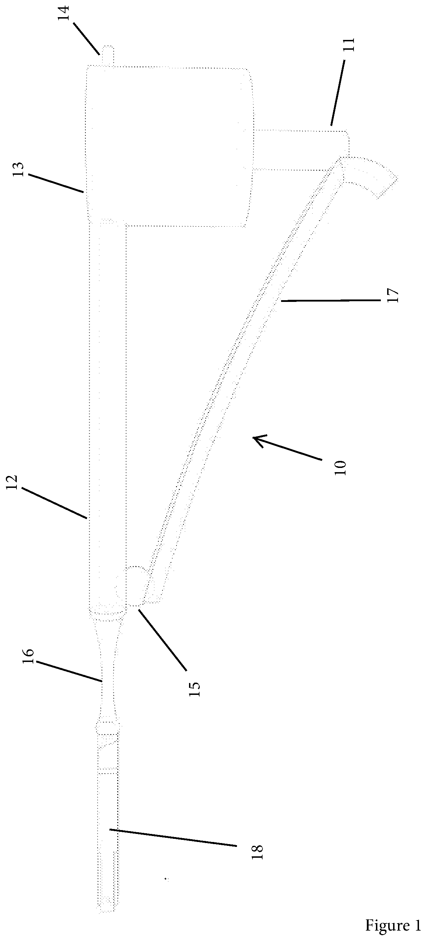

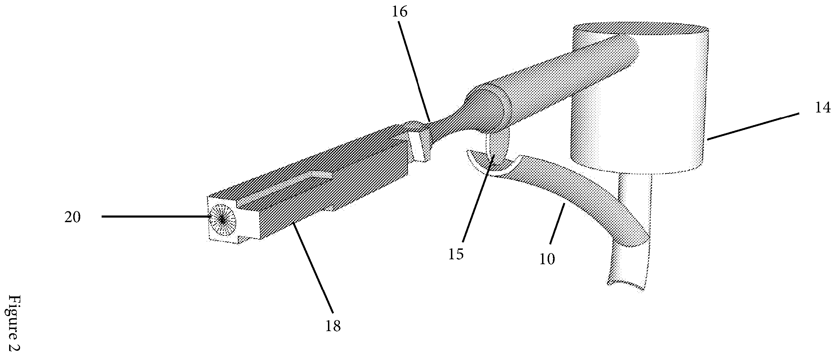

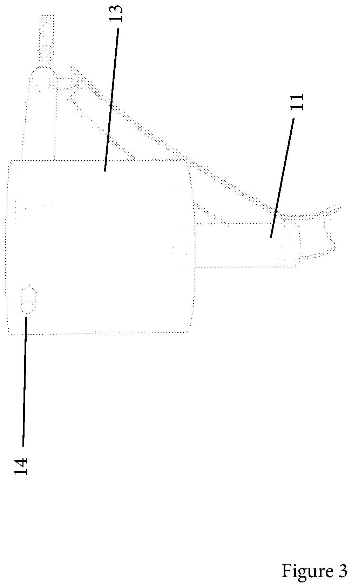

Figures 1, 2 and 3 illustrate external views of an implementation of the present invention. Device 10 includes a liquid chamber 13, connected to a stem 12. Stem includes an internal liquid path (not visible) to the spray head 20. Adjacent to spray head 20 is a fitting 18 for receiving a disposable scalpel blade (not shown).

Device 10 further includes a handle 17, connected at one end via hinge 15 to stem 12, and at the other to plunger 11. Handle 17 preferably has a surface texture to enhance friction and reduce slipping. Similar treatment is preferred for the stem 12. Surgical liquid enters the liquid chamber 13 via barbed fitting 14, which incorporated a one way valve to prevent back pressure. Barbed fitting 14 in use is connected to a flexible tube and then to the supply of surgical liquid. Thus, if handle 17 is depressed, plunger 11 is forced into liquid chamber 13, exerting pressure so that a spray of surgical liquid is produced from spray head 20.

It will be understood that handle 17 preferably provides a resilient return force, so that the handle returns to an outward position once released. This may be provided in any suitable way, for example by the shape and angle of the handle and its interaction with the plunger; a return spring associated with the handle; by the reaction of the liquid system; or by a spring associated with the plunger.

The device 10 may also include additional features. For example, the volume of liquid dispensed by each operation of the handle may be adjustable. In one form, the plunger have an adjustable stroke length, so that the volume displaced is changed, for example by a screw adjustment. In another form, the extent of travel of the handle may be adjustable.

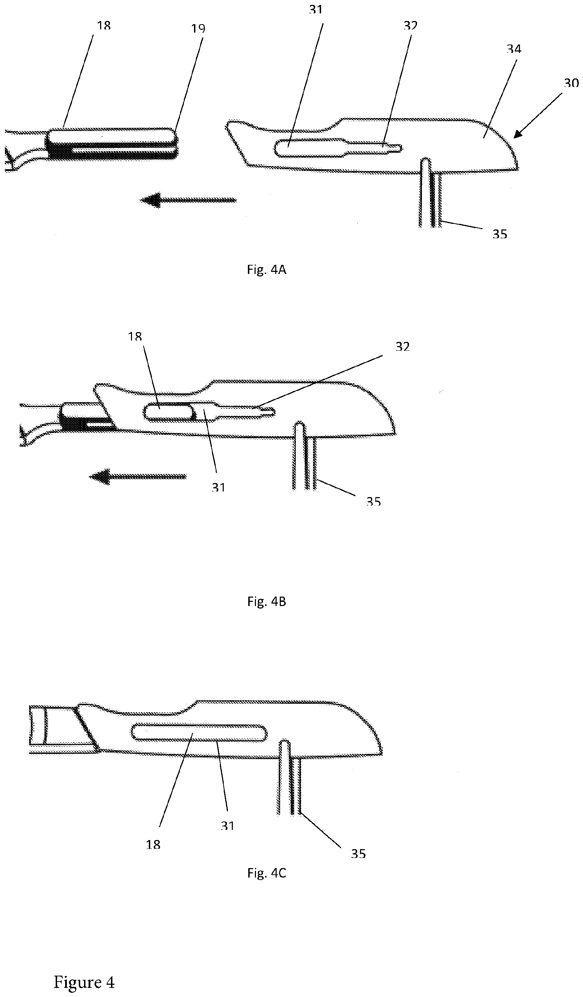

Figures 4A, 4B and 4C illustrate various stages of attaching a blade to the attachment to fitting 18. As can be seen in figure 4A, fitting 18 includes a groove 19, and blade 30 includes an opening 31, with a narrowed section 32 located towards the cutting edge 34.

In figure 4B, the fitting 18 can be seen to pass into opening 31, noting that the blade is somewhat flexible, so that the opening 31 receives fitting 18 and enters groove 19 with the narrowed section 32. The result of the sliding fit is that the fitting 18 and opening 31 are mated, so that the blade is firmly and safely mounted on fitting 18 and hence the device 10. The blade is preferably held with forceps 35 to safely carry out this procedure.

It will be understood that the fitting is specific to the brand and type of blade intended to be used, and the fitting will vary as required for different blades.

In use, the surgeon holds device 10 within one hand, so that the handle 17 can be squeezed by one or more fingers, for example the middle, ring and little fingers. A blade is attached to the fitting 18. The end of handle 17 in this implementation is shaped to prevent the little finger from slipping off. Thus, the surgeon can use the blade to make the necessary incisions, while applying the surgical liquid spray as they wish by selectively operating handle 17. It will of course be understood that different finger arrangements and hand positions are possible with different implementations of the present invention.

The narrowed portion 16 serves to locate the surgeon's forefinger and thumb for operation of the scalpel.

It will be appreciated that this arrangement aligns the spray generally with the scalpel, so that it is easy for the surgeon to understand where the spray will go. In some implementations, an adjustment mechanism (such as a set of screws) could be provided to allow the direction and range of the spray to be adjusted. The spray is preferably aligned so that its axis (assuming the spray is generally conical) is generally parallel to the blade, so that it is clear to the surgeon where the spray will fall relative to the blade.

It will be understood that the spray head may allow for the shape, width, orientation, etc. of the spray to be adjusted as required, to accommodate different procedures, or the preferences of the user.

The handle and release mechanism illustrated is of course only one possible mechanism for release of the surgical liquid. For example, a spring operated release similar to a spray bottle mechanism could be used in a non-pressurised implementation. In a pressurised implementation, for example one where a container is pumped up, a simple release valve may be all that is required to be operated by the handle. The release could take the form of a trigger, a button, or any other suitable mechanism.

The internal passage through stem 12 could take any suitable form. In one implementation if may be a simple tube extending from within the chamber 14 through portion 16 to spray head 20. The passage may be integrally formed with the stem structure, or be a separate tube passing through it.

It will be appreciated that the principle of the present disclosure may be incorporated into various shapes and sizes of device, depending upon the intended use for the scalpel and the associated liquid. For example, the disclosure could be applied to a surgery robot, or to any other mechanism used to hold a blade. The spray head could be incorporated into an entire scalpel, so that the entire scalpel is disposable. The invention is defined by the appended claims.

## Claims

1. A scalpel body (10), including an attachment for a removable blade, the removable blade being adapted to make a surgical incision, a spray head (2 0) adapted to operatively deliver a surgical liquid adjacent to the removable blade and onto the surgical incision as a spray proximal to the removable blade, the spray head (20) is integral with the scalpel body (10) and an external connection for allowing the spray head (20) to be connected to an external supply of surgical liquid, wherein the scalpel body (10) further includes a release mechanism to allow for the selective release of surgical liquid from the spray head; and wherein the spay head (20) is configured so that the surgical liquid is operatively delivered as a spray with an axis generally parallel to the blade.

2. A scalpel body (10) according to claim 1, wherein the scalpel body (10) is connected to the external supply of surgical liquid, and a one way valve is provided to prevent back pressure to the external supply.

3. A scalpel body (10) according to claim 1 or claim 2, further including a removable scalpel blade.

4. A scalpel body (10) according to any one of the preceding claims, further including a handle (17).

## Patentansprüche

1. Skalpellkörper (10) mit einem Aufsatz für eine abnehmbare Klinge, wobei die abnehmbare Klinge zur Durchführung eines chirurgischen Schnittes geeignet ist, einem Sprühkopf (2 0 ), der so beschaffen ist, dass er eine chirurgische Flüssigkeit in der Nähe der abnehmbaren Klinge und auf den chirurgischen Schnitt als Spray in der Nähe der abnehmbaren Klinge abgibt, wobei der Sprühkopf (20) einstückig mit dem Skalpellkörper (10) ausgebildet ist und ein externer Anschluss es ermöglicht, dass der Sprühkopf (20) mit einer externe Zufuhr von chirurgischer Flüssigkeit ist, wobei der Skalpellkörper (10) ferner einen Freigabemechanismus aufweist, um die selektive Freigabe von chirurgischer Flüssigkeit aus dem Sprühkopf zu ermöglichen; und wobei der Sprühkopf (20) so konfiguriert ist, dass die chirurgische Flüssigkeit im Betrieb als ein Spray mit einer Achse im Allgemeinen parallel zur Klinge abgegeben wird.

2. Skalpellkörper (10) nach Anspruch 1, wobei der Skalpellkörper (10) an die externe Versorgung mit chirurgischer Flüssigkeit angeschlossen ist und ein Rückschlagventil vorgesehen ist, um einen Gegendruck zur externen Versorgung zu verhindern

3. Skalpellkörper (10) nach Anspruch 1 oder Anspruch 2, ferner mit einer abnehmbaren Skalpellklinge..

4. Skalpellkörper (10) nach einem der vorhergehenden Ansprüche, ferner mit einem Griff (17).

## Revendications

1. Corps de scalpel (10), incluant une attache pour une lame amovible, la lame amovible étant conçue pour réaliser une incision chirurgicale, une tête de pulvérisation (20) conçue pour délivrer de manière fonctionnelle un liquide chirurgical de manière adjacente à la lame amovible et jusque sur l'incision chirurgicale sous la forme d'un spray proximal à la lame amovible, la tête de pulvérisation (20) est solidaire avec le corps de scalpel (10) et un raccord externe destiné à permettre à la tête de pulvérisation (20) d'être reliée à une alimentation externe de liquide chirurgical, où le corps de scalpel (10) inclut en outre un mécanisme de libération pour permettre la libération sélective de liquide chirurgical depuis la tête de pulvérisation ; et où la tête de pulvérisation (20) est configurée de sorte que le liquide chirurgical est délivré de manière fonctionnelle sous la forme d'un spray avec un axe généralement parallèle à la lame.

2. Corps de scalpel (10) selon la revendication 1, où le corps de scalpel (10) est relié à l'alimentation externe de liquide chirurgical, et une vanne anti-retour est fournie pour empêcher une contre-pression vers l'alimentation externe.

3. Corps de scalpel (10) selon la revendication 1 ou la revendication 2, incluant en outre une lame de scalpel amovible.

4. Corps de scalpel (10) selon l'une quelconque des revendications précédentes, incluant en outre une poignée (17).
